(19) Europäisches Patentamt / European Patent Office / Office européen des brevets

(11) **EP 4 272 775 B1**

(12) **EUROPEAN PATENT SPECIFICATION**

(45) Date of publication and mention
of the grant of the patent:
**08.07.2026 Bulletin 2026/28**

(21) Application number: **22742699.6**

(22) Date of filing: **21.01.2022**

(51) International Patent Classification (IPC):
**A61L 31/18** (2006.01)    **A61L 31/06** (2006.01)
**A61L 31/14** (2006.01)    **A61L 31/16** (2006.01)

(52) Cooperative Patent Classification (CPC):
(C-Sets available)
**A61L 24/0015; A61L 24/0042; A61L 24/043;**
**A61L 24/046;** A61L 2300/22; A61L 2300/44;
A61L 2400/06; A61L 2430/36          (Cont.)

(86) International application number:
**PCT/JP2022/002266**

(87) International publication number:
**WO 2022/158580 (28.07.2022 Gazette 2022/30)**

(54) **EMBOLIC MATERIAL AND METHOD FOR PRODUCING SAME**

EMBOLIEMATERIAL UND VERFAHREN ZUR HERSTELLUNG DAVON

MATÉRIAU EMBOLIQUE ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.01.2021 JP 2021008824**

(43) Date of publication of application:
**08.11.2023 Bulletin 2023/45**

(73) Proprietors:
• **Tokai University Educational System**
  **Tokyo, 151-0063 (JP)**
• **Keio University**
  **Tokyo, 108-8345 (JP)**

(72) Inventors:
• **HASEBE, Terumitsu**
  **Hachioji-shi, Tokyo 192-0032 (JP)**
• **OKAMOTO, Yutaka**
  **Yokohama-shi, Kanagawa 223-8522 (JP)**
• **BITO, Kenta**
  **Hachioji-shi Tokyo 192-0032 (JP)**
• **HOTTA, Atsushi**
  **Yokohama-shi, Kanagawa 223-8522 (JP)**

(74) Representative: **Swindell & Pearson Limited**
**48 Friar Gate**
**Derby DE1 1GY (GB)**

(56) References cited:
**WO-A1-2018/151214      CN-B- 101 108 262**
**JP-A- 2007 325 910**

• **OKAMOTO YUTAKA ET AL: "Fabrication of**
**radiopaque drug-eluting beads based on**
**Lipiodol/biodegradable-polymer for image-**
**guided transarterial chemoembolization of**
**unresectable hepatocellular carcinoma",**
**POLYMER DEGRADATION AND STABILITY,**
**BARKING, GB, vol. 175, 24 February 2020**
**(2020-02-24), XP086131931, ISSN: 0141-3910,**
**[retrieved on 20200224], DOI: 10.1016/**
**J.POLYMDEGRADSTAB.2020.109106**
• **KIM MI RI, LEE SEUNGWOO, PARK JUNG-KI,**
**CHO KUK YOUNG: "Golf ball-shaped PLGA**
**microparticles with internal pores fabricated by**
**simple O/W emulsion", CHEMICAL**
**COMMUNICATIONS, ROYAL SOCIETY OF**
**CHEMISTRY, UK, vol. 46, no. 39, 1 January 2010**
**(2010-01-01), UK**
**, pages 7433 - 7435, XP055953110, ISSN:**
**1359-7345, DOI: 10.1039/c0cc01738h**

- TAKU TAKAMI, MURAKAMI YOSHIHIKO: "Development of PEG–PLA/PLGA microparticles for pulmonary drug delivery prepared by a novel emulsification technique assisted with amphiphilic block copolymers", COLLOIDS AND SURFACES B: BIOINTERFACES, AGRICULTURE SCIENCES - FOOD SCIENCE AND TECHNOLOGY, vol. 87, no. 2, 1 October 2011 (2011-10-01), pages 433 - 438, XP055021206, ISSN: 09277765, DOI: 10.1016/j.colsurfb.2011.06.004
- JEON SEONG IK, KIM MOO SONG, KIM HYUNG JUN, KIM YOUNG IL, JAE HWAN JUN, AHN CHEOL-HEE: "Biodegradable poly(lactide-co-glycolide) microspheres encapsulating hydrophobic contrast agents for transarterial chemoembolization", JOURNAL OF BIOMATERIALS SCIENCE. POLYMER EDITION., VSP, UTRECHT., vol. 33, no. 4, 4 March 2022 (2022-03-04), NL
, pages 409 - 425, XP009538450, ISSN: 0920-5063, DOI: 10.1080/09205063.2021.1990472

(52) Cooperative Patent Classification (CPC): (Cont.)

C-Sets
A61L 24/043, C08L 67/04;
A61L 24/046, C08L 67/04

**Description**

CROSS-REFERENCE TO RELATED APPLICATION

This international application claims the benefit of Japanese Patent Application No. 2021-008824 filed on January 22, 2021 with the Japan Patent Office.

TECHNICAL FIELD

The present disclosure relates to an embolic material and a method for producing the same.

BACKGROUND ART

In transcatheter arterial chemoembolization (TACE) which is a treatment method of hepatocellular carcinoma, a thin tubular catheter is inserted from a femoral artery or a brachial/radial artery to an artery (mainly, a hepatic artery) that supplies nutrients to a tumor, and anticancer drug-eluting embolization beads (Drug-Eluting Beads: DEBs) are injected. This stops the blood flow that supplies nutrients to the tumor, and causes necrosis of the tumor due to antitumor effect of the anticancer drug. Injection of DEBs is performed under X-ray fluoroscopy. However, normal DEBs themselves have no X-ray visibility, which makes it difficult to grasp the embolized state (Non-Patent Document 1). In addition, when DEBs flow into an unintended blood vessel and embolize the blood vessel, serious complications may occur. Also, since an unintended embolized site cannot be confirmed by X-rays, it is difficult to predict the onset of complications. Further, since the injected DEBs remain permanently in the living body, there is concern about an increased risk of complications. Moreover, it is desirable that DEBs as such are uniform in size and spherical in order to selectively embolize only the target blood vessel.

Therefore, in TACE, DEBs have been desired which have a uniform particle size and satisfy both X-ray visibility and a property of not remaining permanently in the living body (Non-Patent Documents 2 and 3).

Patent Document 1 discloses an embolic material containing a biodegradable polymer and a liposoluble contrast agent. However, it was considered that the embolic material of Patent Document 1 might have insufficient uniformity in particle diameter.

Patent Document 2 discloses a method of preparing an embolic material using a copolymer consisting of a water-soluble polymer and a biodegradable polymer. However, it was considered that the embolic material as such might not be spherical and might have insufficient formability.

PRIOR ART DOCUMENTS

PATENT DOCUMENTS

Patent Document 1: Japanese Unexamined Patent Application Publication No. 2018-130322
Patent Document 2: Japanese Unexamined Patent Application Publication No. 2004-167229

NON-PATENT DOCUMENTS

Non-Patent Document 1: R. Duran, et al. Theranostics, 6, 28-39, 2016
Non-Patent Document 2: N. Richard, et al. Case Reports in Gastroenterology, 12(2), 352-9, 2018
Non-Patent Document 3: A. Toro, et al. Case Reports in Surgery, 2015.

SUMMARY OF THE INVENTION

PROBLEMS TO BE SOLVED BY THE INVENTION

It is desirable that the present disclosure provides an embolic material having a more uniform particle size, better formability, X-ray visibility and biodegradability.

MEANS FOR SOLVING THE PROBLEMS

The invention is defined by the claims.

BRIEF DESCRIPTION OF THE DRAWINGS

FIG. 1 is a diagram schematically showing a method for preparing polylactic acid (PLA)/ poly(lactic-co-glycolic acid) (PLGA)/Lipiodol (LPD) beads by O/W emulsion method.

FIG. 2 shows tomographic CT images immediately after embolization when PLA/PLGA/LPD beads (PLA:PLGA:LPD=20:20:60, and PLA:PLGA:LPD=40:0:60) are injected into a rabbit hepatic artery.

FIG. 3 shows vascular CT images before and immediately after embolization, one week after embolization (PLA:PLGA:LPD=20:20:60) and one week and two weeks after embolization (PLA:PLGA:LPD=40:0:60) when the PLA/PLGA/LPD beads (PLA:PLGA:LPD=20:20:60, and PLA:PLGA:LPD=40:0:60) are injected into the rabbit hepatic artery.

FIG. 4 is a graph showing weight reduction of the PLA/PLGA/LPD beads (PLA:PLGA:LPD=20:20:60, PLA:PLGA:LPD=30:10:60, and PLA:PLGA:LPD=40:0:60) over time after addition of a degrading enzyme (Proteinase K). A horizontal axis shows time elapsed after addition of the degrading enzyme, and a vertical axis shows a weight reduction rate (%).

FIG. 5 shows graphs showing biodegradability of the PLA/PLGA/LPD beads (PLA:PLGA:LPD=20:20:60, PLA:PLGA:LPD=30:10:60, and PLA:PLGA:LPD=40:0:60) over time after addition of the degrading enzyme (Proteinase K), using a molecular weight ratio as an index. The horizontal axis shows time elapsed after addition of the degrading enzyme, and the vertical axis shows the molecular weight ratio.

FIG. 6 is a diagram showing a typical example of chemical shifts of the PLA/PLGA/LPD beads measured by nuclear magnetic resonance (NMR) method.

FIG. 7 shows graphs showing weight ratios of PLA, PLGA and LPD in the PLA/PLGA/LPD beads (PLA:PLGA:LPD=20:20:60, PLA:PLGA:LPD=30:10:60, and PLA:PLGA:LPD=40:0:60) over time after addition of the degrading enzyme (Proteinase K). The horizontal axis shows time elapsed after addition of the degrading enzyme, and the vertical axis shows the weight ratios (%).

FIG. 8 shows images of PLA/LPD beads (PLA:LPD=1.0:0.5, PLA:LPD=1.0:1.0, PLA:LPD=1.0:1.5, PLA:LPD=1.0:2.0, PLA:LPD=1.0:3.0) taken by X-ray micro-CT.

FIG. 9 shows images of bead cross sections of the PLA/PLGA/LPD beads (PLA:PLGA:LPD=20:20:60, and PLA:PLGA:LPD=40:0:60) observed by the X-ray micro-CT.

FIG. 10 shows images of the PLA/PLGA/LPD beads (PLA:PLGA:LPD=0:40:60, PLA:PLGA:LPD=10:30:60, PLA:PLGA:LPD=20:20:60, PLA:PLGA:LPD=30:10:60, and PLA:PLGA:LPD=40:0:60) observed by a scanning electron microscope (SEM), and a diagram showing amounts of PLA, PLGA and LPD charged at the time of preparing the respective beads and amounts of PLA, PLGA and LPD measured by NMR after bead preparation.

FIG. 11 shows images obtained by analyzing bead cross sections of the PLA/PLGA/LPD beads (PLA:PLGA:LPD=20:20:60, PLA:PLGA:LPD=30:10:60, and PLA:PLGA:LPD=40:0:60) with an electron microscope image. (a) to (c) respectively show 1000x magnified images of beads with PLA:PLGA:LPD=20:20:60, PLA:PLGA:LPD=30:10:60, and PLA:PLGA:LPD=40:0:60, and (d) to (f) respectively show 2000x magnified images of beads with PLA:PLGA:LPD=20:20:60, PLA:PLGA:LPD=30:10:60, and PLA:PLGA:LPD=40:0:60.

FIG. 12A shows images of surface structures of the PLA/PLGA/LPD beads (PLA:PLGA:LPD=20:20:60, PLA:PLGA:LPD=30:10:60, and PLA:PLGA:LPD=40:0:60) immediately after addition of the degrading enzyme (Proteinase K) and 14 days after addition of the degrading enzyme (Proteinase K) observed by SEM.

FIG. 12B shows images of surface structures of the PLA/PLGA/LPD beads (PLA:PLGA:LPD=20:20:60, PLA:PLGA:LPD=30:10:60, and PLA:PLGA:LPD=40:0:60) 21, 28, and 40 days after addition of the degrading enzyme (Proteinase K) observed by SEM.

FIG. 13 is a schematic diagram showing a generation mechanism of the PLA/PLGA/LPD beads.

FIG. 14 shows graphs showing particle size distributions of the respective PLA/LPD beads prepared by different rotation speeds of agitation (500 rpm, 300 rpm and 200 pm). A horizontal axis of each graph shows the particle diameter, and a vertical axis shows the frequency (%).

FIG. 15 is a graph showing particle size distributions of the respective beads with different Qc/Qd ratios (1500, 2000, 2500 and 3000) prepared by a microchannel method. A horizontal axis of each graph shows the particle size of the bead, and a vertical axis shows the frequency (%).

FIG. 16 shows images of PLGA/LPD beads (PLGA (LA:GA=50:50):PLGA (LA:GA=75:25):LPD=40:0:60, PLGA (LA:GA=50:50):PLGA (LA:GA=75:25):LPD=10:30:60, and PLGA (LA:GA=50:50):PLGA (LA:GA=75:25):LPD=0:40:60) observed by SEM.

## MODE FOR CARRYING OUT THE INVENTION

An example embodiment of the present disclosure will be described hereinafter with reference to the drawings.

### 1. Embolic material

An embolic material of the present disclosure can be obtained by dispersing a hydrophobic mixture containing a specific material in an aqueous solvent (O/W emulsion method).

Specifically, the embolic material of the present disclosure can be obtained by a production method comprising a step for dispersing a mixture containing a polymer having biodegradability (hereinafter, biodegradable polymer), an oil-based contrast agent and a solvent in a hydrophilic liquid. The mixture is supplied to the hydrophilic liquid, thereby being dispersed in particulate form in the hydrophilic liquid. The particulate mixture gradually volatilizes the solvent in the hydrophilic liquid, and eventually becomes solidified or semi-solidified.

By the aforementioned production method of the embolic material, an embolic material having a more uniform particle size, better formability, X-ray visibility and biodegradability can be produced.

An embolic material comprising only a hydrophilic biodegradable polymer easily loses its shape and is difficult to maintain a specific shape. In contrast, according to the production method of the embolic material of the present disclosure, the embolic material produced comprises an oil-based contrast agent, a hydrophobic biodegradable polymer, or a hydrophobic biodegradable polymer and a hydrophilic biodegradable polymer. The embolic material is formed into a spherical shape by combining these components. Thus, the embolic material of a desired particle size can be easily obtained, and can be reliably retained in the target vascular portion.

When there is a large variation in particle size of the embolic material, a blood flow rate can be reduced by intravascular administration of the embolic material, but it is difficult to stop the blood flow. In contrast, the embolic material of the present disclosure has a more uniform particle size. Therefore, the embolic material can be retained in the target vascular portion and the blood flow can be almost certainly stopped. In addition, since the embolic material contains a contrast agent, the embolic material can be retained in the target vascular portion, and angiography can be performed.

When the mixture containing the oil-based contrast agent and the solvent is dispersed in the hydrophilic liquid, the particle size of the embolic material of the present disclosure can be adjusted as required by means of such as adjusting an agitation rate of the hydrophilic liquid. Therefore, the particle size of the embolic material can be adjusted in accordance with the thickness of the vascular portion where it is desired the embolic material stays.

The embolic material of the present disclosure comprises the biodegradable polymer. Thus, the embolic material, after administered into a living body, is biodegraded in the living body, and is excreted from the body.

The embolic material of the present disclosure is produced using O/W emulsion method, and thus can be produced at 0°C to 50°C (for example, normal temperature). Hereinafter, the production method of the embolic material of the present disclosure will be described in detail.

In the present disclosure, "biodegradability" means a property of being degraded into bioabsorbable monomers or oligomers by hydrolysis in a living body. When a polymer has biodegradability, the polymer is hydrolyzed in the living body, and the effects on normal tissue caused by the embolic material remaining in the living body can be reduced. Also, when a polymer has biodegradability, the embolic material is hydrolyzed in the living body, and thus the embolic material can be safely used repeatedly in the same subject.

Examples of the biodegradable polymer include biodegradable polyesters, biodegradable proteins, biodegradable polysaccharides, and biodegradable polyamino acids. The preferable biodegradable polymer is a biodegradable polyester. Since the biodegradable polymer is a biodegradable polyester, the degradation rate can be adjusted over a wide period of time, from days to years.

The degree of polymerization of the biodegradable polymer is not particularly limited, and can be adjusted as required. As an example, the degree of polymerization of the biodegradable polymer may be 50 to 6000.

The biodegradable polymer has at least one type of hydrophobic biodegradable polymer (hereinafter, hydrophobic polymer). In the present disclosure, "hydrophobic" means a property of being insoluble in water and having extremely low water absorption.

Examples of the hydrophobic polymer include polylactic acid (PLA), polycaprolactone (PCL), polybutylene succinate (PBS), and polydioxanone (PDS). When the biodegradable polymer is hydrophobic, an embolic material that has a spherical shape and has high X-ray visibility can be obtained.

The content of the hydrophobic polymer, for example, when the content of the embolic material is 100% by mass, may be 10 to 50% by mass or more, and preferably 20 to 40% by mass. This allows production of an embolic material that has good formability and exhibits sufficient X-ray visibility.

It is preferable that the weight average molecular weight of the hydrophobic polymer is 5,000 to 200,000. As an example, in a case of PLA, it is preferable that the weight average molecular weight is 150,000 or more. This allows the hydrophobic polymer to hold the oil-based contrast agent and form the embolic material into a spherical shape.

In addition, as the hydrophobic polymer, a copolymer having a hydrophobic monomer and a hydrophilic monomer as constituent units (hereinafter, copolymer) can be used. Examples of the hydrophobic monomer include lactic acids, and caprolactones. Examples of the hydrophilic monomer includes glycolic acids. Specifically, examples of the copolymer include poly(lactic-co-glycolic acid) (PLGA) or caprolactone-co-glycolic acid (PCGA).

The copolymer can become hydrophobic as a whole as the proportion of hydrophobic monomers increases, and conversely the copolymer can become hydrophilic as a whole as the proportion of hydrophilic monomers increases. In order for the copolymer to function as a hydrophobic polymer, the amount of hydrophobic monomers in the copolymer should be greater than the amount of hydrophilic monomers. In order for the copolymer to function as a hydrophobic polymer, the molar ratio of hydrophobic monomers to hydrophilic monomers in the copolymer is preferably 55:45 to 100:0, more preferably 60:40 to 90:10, more preferably 65:35 to 85:15, even more preferably 70:30 to 80:20, and most preferably 75:25.

The biodegradable polymer may comprise, in addition to the aforementioned hydrophobic polymer, at least one type of hydrophilic biodegradable polymer (hereinafter, hydrophilic polymer). In the present disclosure, "hydrophilic" means a property of being soluble or miscible in water, or a property of being insoluble in water and having high water absorption.

In the aforementioned copolymer, when the molar ratio of the hydrophobic monomer to the hydrophilic monomer is 50:50 to 0:100, the copolymer functions as a hydrophilic polymer.

Therefore, the embolic material of the present disclosure may comprise, as a biodegradable polymer, a copolymer containing one or more types of hydrophobic monomers and hydrophilic monomers. Specifically, in the embolic material of the present disclosure, the biodegradable polymer may have a copolymer of which molar ratio is adjusted so that the copolymer functions as a hydrophobic polymer. Also, in the embolic material of the present disclosure, the biodegradable polymer may have both a copolymer of which molar ratio is adjusted so that the copolymer functions as a hydrophobic polymer, and a copolymer of which molar ratio is adjusted so that the copolymer functions as a hydrophilic polymer.

The weight average molecular weight of the aforementioned copolymer may be 5,000 to 200,000.

It is preferable that the aforementioned copolymer is a random copolymer. In random copolymers, hydrophilic portions deriving from a hydrophilic monomer and hydrophobic portions deriving from a hydrophobic monomer are randomly arranged. Since random copolymers are easily degraded at the hydrophilic portions during biodegradation, the hydrophobic portions existing between the hydrophilic portions are also finely degraded due to degradation of the hydrophilic portions. Thus, random copolymers are more finely degraded than copolymers in other forms (for example, block copolymers). Generally, in crystals in which the molecular chains are regularly arranged, for example, those as formed in block copolymers, water molecules are less likely to enter between the molecular chains. On the other hand, due to random copolymerization of copolymers, regularity of molecular chains is lost, and it is difficult for crystals to be formed. Therefore, the degradation rate of random copolymers is faster than copolymers in other forms (for example, block copolymer). From the above, since the copolymer is a random copolymer, degradability of the embolic material in the living body can be improved.

When the aforementioned copolymer is a hydrophilic polymer, it is preferable that the hydrophilic polymer has a portion of the structure of a hydrophobic polymer in order to improve compatibility with the hydrophobic polymer in the embolic material. For example, if the hydrophobic polymer is PLA, it is preferable that the hydrophilic polymer is PLGA containing LA (lactic acid). Also, if the hydrophobic polymer is PCL, it is preferable that the hydrophilic polymer is PCGA containing CL (caprolactone).

Here, it is desired that the embolic material loses its function in a specified period of time (for example, approximately a week) after vascular embolization and the blood flow is restored. However, a simple hydrophobic polymer having a large molecular weight may take one to two years to be completely degraded in the living body. Therefore, in the embolic material, by mixing a hydrophilic polymer in addition to a hydrophobic polymer, the degradation rate in the living body can be relatively fast. Also, by increasing a compound ratio of the hydrophilic polymer to the hydrophobic polymer, the degradation rate in the living body can be faster. As a result, it is considered that the blood flow can be restored quickly.

When one type of hydrophobic polymer and one type of hydrophilic polymer are used as biodegradable polymers, the weight mixing ratio of one polymer to the other polymer may be 3.0 or less, as an example.

In the present disclosure, as an example, PLA which is a hydrophobic polymer and/or PLGA which is a hydrophilic polymer may be selected as the biodegradable polymer(s). PLA and PLGA have been used in blood-contacting medical devices such as stents, and are known to have high biocompatibility. When PLA and PLGA are selected as biodegradable polymers, the weight mixing ratio of PLA to PLGA is preferably 1.0 or more, and more preferably 1.0 to 3.0. Generally, the degradation rate of PLGA is faster than PLA which is a crystal polymer. Therefore, by using these two biodegradable polymers, the degradation rate of the embolic material can be controlled. In addition, since the weight mixing ratio of PLA to PLGA is 1.0 to 3.0, an embolic material that has good formability (that is, has a spherical shape) and exhibits sufficient X-ray visibility can be obtained.

An oil-based contrast agent is used in order to form the embolic material. By specifically using an oil-based contrast agent among contrast media, the mixture is dispersed in particulate form in the hydrophilic liquid, and thus an embolic material that has good formability and high visibility under X-ray fluoroscopy can be produced.

The weight mixing ratio of the oil-based contrast agent to the biodegradable polymer in the embolic material is preferably 1.0 to 2.0, and most preferable weight mixing ratio is 1.5. Here, when the weight mixing ratio of the oil-based contrast agent to the biodegradable polymer is too small, it may be difficult to produce an embolic material that exhibits sufficient X-ray visibility. On the other hand, when the weight mixing ratio of the oil-based contrast agent to the biodegradable polymer is too large, X-ray visibility of the embolic material may decrease due to such as phase separation of the excess oil-based contrast agent that is not fully miscible with the biodegradable polymer.

Therefore, since the weight mixing ratio of the oil-based contrast agent to the biodegradable polymer is in a range of 1.0 to 2.0, an embolic material that has good formability (that is, has a spherical shape) and exhibits sufficient X-ray visibility can be produced.

It is preferable that the oil-based contrast agent is swelled at a molecular level in a biodegradable polymer network. Examples of the oil-based contrast agent include ethyl ester of iodinated poppy-seed oil fatty acid. Examples of commercially available ethyl ester of iodinated poppy-seed oil fatty acid include Lipiodol (LPD) (registered trademark). In the present disclosure, Lipiodol is preferable as the oil-based contrast agent to be used, from the viewpoint of high hepatic tumor accumulation and local retention of tumors. The oil-based contrast agent may be other than ethyl ester of iodinated poppy-seed oil fatty acid.

The oil-based contrast agent is retained inside the embolic material. Specifically, since the oil-based contrast agent exists inside the embolic material, the contrast imaging function is exhibited until the embolic material is completely degraded in the living body. Therefore, for example, as compared to a case where the oil-based contrast agent adheres to the surface of the embolic material, the contrast imaging function can be exhibited in the long term.

A pharmaceutical drug may be further mixed in the aforementioned mixture. The pharmaceutical drug may be dissolved, for example, in the oil-based contrast agent. In this case, the oil-based contrast agent may be compatible with the pharmaceutical drug. In addition, the oil-based contrast agent and the pharmaceutical drug may be maintained without affecting each other's properties. When the mixture further contains a pharmaceutical drug, the embolic material when produced may have a drug controlled release property. Examples of the pharmaceutical drug include anticancer drug, anti-inflammatory drug, and antifungal drug. Examples of the anticancer drug include injectable miriplatin hydrate, tacrolimus, cyclosporine, mizoribine, cyclophosphamide, azathioprine, and mycophenolate mofetil. An example of a commercial product of the injectable miriplatin hydrate is Miripla (registered trademark). Examples of the anti-inflammatory drug include steroids, prednisolone, loxoprofen, acetaminophen, ibuprofen, and aspirin. Examples of antibacterial/antifungal drug include rifampicin, isoniazid, ethambutol, pyrazinamide, streptomycin, amikacin, levofloxacin, imipenem, meropenem, piperacillin tazobactam, clindamycin, voriconazole, amphotericin B, micafungin, caspofungin, fluconazole, and itraconazole.

As the hydrophilic liquid, for example, an aqueous solution in which an emulsifier is dissolved can be used. Use of the aqueous solution in which the emulsifier is dissolved allows the mixture to remain particulate in the aqueous solution. Examples of the emulsifier include anionic surfactants such as fatty acid sodium, monoalkyl sulfates, alkylpolyoxyethylene sulfates, alkylbenzenesulfonates, and monoalkyl phosphates (e.g., sodium lauryl sulfate), cationic surfactants such as alkyltrimethylammonium salts, dialkyldimethylammonium salts, and alkylbenzyldimethylammonium salts (e.g., distearyldimethylammonium chloride, and benzalkonium chloride), amphoteric surfactants such as amine oxides, and betaine (e.g., cocamidopropyl betaine), and nonionic surfactants such as polyoxyethylene alkyl ethers, fatty acid sorbitan esters, alkyl polyglucosides, fatty acid diethanolamides, alkyl monoglyceryl ethers, polyethylene glycol, and polyvinyl alcohol. Polyvinyl alcohol (PVA) is preferable due to its comparatively high biocompatibility. As an example, when a PVA aqueous solution is used as the hydrophilic liquid, a concentration of the PVA aqueous solution may be within a range of 0.1 to 5 w/v%, and preferably from 0.4 to 0.6 w/v%.

The solvent may be an organic solvent. It is preferable that the organic solvent can dissolve the oil-based contrast agent and the biodegradable polymer, is hydrophobic, and is volatile with low boiling point. The boiling point of the organic solvent to be used may be 100°C or less, may be 20 to 80°C, and may be 40 to 60°C. For example, halogenated hydrocarbon such as dichloromethane (boiling point 40°C) or chloroform (boiling point 60°C) is used as the organic solvent.

As a means to supply the aforementioned mixture to the hydrophilic liquid, for example, a syringe, a nozzle, and the like may be used to supply the mixture to the hydrophilic liquid by dripping. Also, as a means to supply the mixture to the hydrophilic liquid, a syringe, a nozzle, and the like may be used to supply the mixture to the hydrophilic liquid by injection. Diameters of openings of the syringe, nozzle, and the like to be used at this time are preferably within a range of 0.01 to 0.9 mm. By adjusting the diameters of the openings, the particle diameter of the embolic material to be produced can be varied.

The form of the mixture supplied to the hydrophilic liquid is not limited and various forms can be taken. However, the preferable form is spherical.

The step for dispersing the mixture containing the oil-based contrast agent and the solvent in the hydrophilic liquid may be performed by supplying the mixture to the hydrophilic liquid while agitating the hydrophilic liquid. The agitation may be performed, for example, using a stirrer (SM-102, manufactured by AS ONE Corporation). Agitation may be performed, for example, using a stirring bar. The stirring bar can have any selectable shape and size. However, it is preferable that agitation is performed using a stirring bar having a shape that makes it easy to increase the shearing force of the hydrophilic liquid by agitation. As an example, a propeller shaped stirring bar with stirrer blades of 50 mm in diameter may be used.

The rotation speed of the stirrer in a case of using the aforementioned stirrer can be set within a range of 100 to 1000 rpm. In order to prepare spherical particles, it is preferable that the rotation speed is a speed at which at least the dispersed mixture does not precipitate. Specifically, it is preferable that the rotation speed is 200 to 500 rpm. The particle size of the embolic material can be adjusted by changing the rotation speed of the stirrer. Specifically, when the rotation speed is increased, the shearing force of the hydrophilic liquid is increased, and the particle size of the embolic material is decreased. Temperature of the hydrophilic liquid during agitation may be 0°C to 50°C, and preferably 10°C to 30°C, and more preferably normal temperature. Agitation time can be set within a range of 0.5 to 48 hours, and preferably 15 to 20 hours.

The embolic material that has remained after volatilization of the solvent may be dried after collected. Collection may be performed, for example, by filtration under reduced pressure. Drying time can be set within a range of 0.5 to 48 hours, and preferably 18 to 36 hours. Drying temperature may be 0°C to 50°C, and preferably normal temperature.

The embolic material obtained by the aforementioned production method has a biodegradable polymer and an oil-based contrast agent. The embolic material obtained by the aforementioned production method may consist of a biodegradable polymer and an oil-based contrast agent.

The embolic material obtained by the aforementioned production method has depressions (dimples) on its surface. The depressions are uniformly formed on the surface of the embolic material, and can have a hemispherical shape. By increasing the content of the hydrophobic polymer in the embolic material, the number of the depressions increase and the diameter of each depression can decrease.

The dimples on the bead surface can be formed by phase separation between the mixture and the solvent caused during formation of the beads in the O/W emulsion method. Specifically, excess oil-based contrast agent causes phase separation during removal of the solvent and leaks to the hydrophilic liquid. The dimples can be formed on the bead surface as the traces.

The embolic material may be porous having voids on its surface and inside. The voids can be generated as traces of excess oil-based contrast agent that has caused phase separation during removal of the solvent and leaked to the hydrophilic liquid. As the content of the hydrophobic polymer in the embolic material increases, these voids increase in number and the diameter can decrease. In addition, this void structure can make it easy for the embolic material to degrade. Therefore, the blood flow after vascular embolization can be restored.

Such a porous shape is difficult to observe in embolic materials obtained by other production methods in which the volatilization speed of the solvent is fast. For example, when an embolic material is prepared using a spray drying method, the volatilization speed of the solvent is fast and the solvent volatilizes before phase separation. Thus, it is considered difficult to form a porous embolic material.

In the present disclosure, a more uniformly sized particulate embolic material than before can be obtained.

The size of the embolic material has no specific limitation, and can be adjusted as required. As an example, the embolic material may have an average particle size of 50 to 1000 μm, and the preferable average particle size is 100 to 500 μm. When the average particle size is 50 μm or more and less than 150 μm, the standard deviation may be 5 to 30 μm. When the average particle size is 150 μm or more and less than 300 μm, the standard deviation may be 30 to 60 μm. When the average particle size is 300 μm or more and less than 500 μm, the standard deviation may be 60 to 100 μm. When the average particle size is 500 μm or more and less than 1000 μm, the standard deviation may be 100 to 200 μm.

The average particle size may be an average value of diameters of respective image data obtained by imaging a specific number of embolic materials with a microscope or the like and measured by analysis software. The specific number may be 20 or more, 50 or more, 100 or more, and 150 or more.

The embolic material of the present disclosure is in the form of more uniformly sized particles than before. Thus, only the target blood vessel can be selectively embolized.

2. Usage of embolic material

The embolic material can be used for medical purposes. The embolic material may be injected into a blood vessel through a catheter to temporarily stop the blood flow, and used to visualize the embolized site in the blood flow with X-ray irradiation.

The embolic material may be used for treatment of a subject. Examples of the subject include, but are not limited to, mammals, for example, primates such as humans and chimpanzees, laboratory animals such as rats, mice, and rabbits, livestock animals such as pigs, cows, horses, and sheep, and companion animals such as dogs and cats. The preferable subject is humans.

Specifically, the embolic material can be used, for example, in TACE for treatment of hepatocellular carcinoma. When the embolic material contains a pharmaceutical drug, the embolic material can be used as, for example, DEB in TACE. The embolic material can embolize, for example, the hepatic artery.

In addition to rheumatoid arthritis, chronic inflammation of joints and muscles is caused by neovessels formed abnormally by branching from existing blood vessels. It is known that such chronic inflammation can be treated by injecting a drug combination of imipenem hydrate and cilastatin sodium (antibiotic) into the neovessels using a catheter (Y.Okuno, et al. J.Vasc. Interv. Radiol., 24, 787-792, 2013). Such treatment is known as Transcatheter Arterial Micro Embolization. The embolic material can be used, for example, in such a Transcatheter Arterial Micro Embolization.

The embolic material can be also used in, for example, bronchial artery embolization (BAE) to treat hemoptysis and embolization for various other systemic hemorrhages and hypervascular tumors.

[Examples]

(Example 1) Preparation of samples

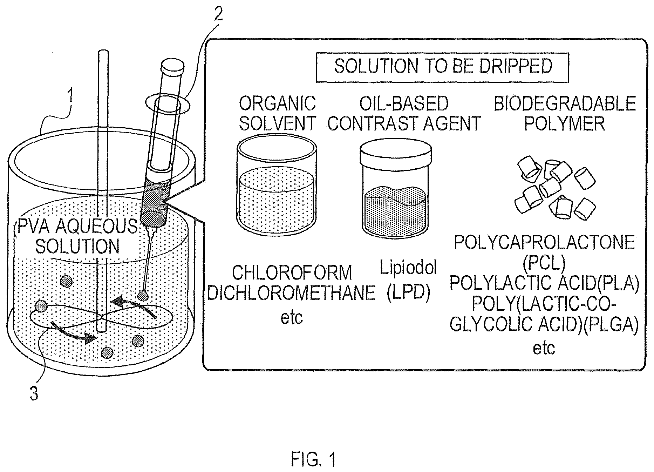

PLA/PLGA/LPD beads were prepared by the O/W emulsion method (see FIG. 1). The specific preparation procedure is shown below. In the following examples, "bead" means embolic material.
1) Biodegradable polymer (PLA (Mw=207, 000, manufactured by Nature Works) and PLGA (poly(D, L-lactide-co-glicolide), product name: RESOMER RG502, Mw=7,000 to 17,000, LA:GA=50:50, random copolymer, manufactured by Sigma-Aldrich)) and LPD (brand name: Lipiodol 480 injection 10 mL, manufactured by Guerbet Japan) were weighed into a screw bottle with an electronic balance (product name: GR-60, manufactured by A&D Company, Limited) according to the composition shown in Table 1 below, then added with 10 mL of DCM, and dissolved at 35°C and a rotation speed of 300 rpm to obtain a DCM solution.

[Table 1]

| Form. | Dispersed phase | | | | Continuous phase | Agitation rate (rpm) |
|---|---|---|---|---|---|---|
| | PLA (g) | PLGA (g) | LPD (g) | DCM (mL) | PVAaq (w/v%) | |
| 1 | 0 | 0.4 | | | | |
| 2 | 0.1 | 0.3 | | | | |
| 3 | 0.2 | 0.2 | 0.6 | 10 | 0.5 | 300 |
| 4 | 0.3 | 0.1 | | | | |
| 5 | 0.4 | 0 | | | | |

2) 500 mg of PVA (manufactured by Tokyo Chemical Industry Co., Ltd., molecular weight: approximately 70,000) was weighed with the electronic balance, and added to 1 L of water weighed with a measuring cylinder. The mixture was agitated at 50°C and 500 rpm to form a 0.5 w/v% PVA aqueous solution.
3) As shown in FIG. 1, 500 mL of PVA aqueous solution weighed with the measuring cylinder was placed in 1 L beaker 1. Using a syringe 2 with a 20 gauge needle, the DCM solution prepared in 1) was injected therein at normal temperature while agitating the DCM solution at a rotation speed of 300 rpm using a stirrer 3 (manufactured by AS ONE Corporation, SM-102). Thereafter, the PVA aqueous solution was further agitated using the stirrer 3 (manufactured by AS ONE Corporation, SM-102) at a rotation speed of 300 rpm for 18 hours to volatilize DCM, thereby preparing PLA/PLGA/LPD beads. Stirrer blades (tornado stirrer blades manufactured by AS ONE Corporation, diameter 50 mm) of the stirrer 3 were set at the bottom of the beaker 1, and rotated so that the PVA aqueous solution in the beaker 1 produced a tornado.
4) The PLA/PLGA/LPD beads were collected by filtering the solution containing the PLA/PLGA/LPD beads through a glass filter paper (manufactured by ADVANTEC TOYO KAISHA, LTD., GS-25). The PLA/PLGA/LPD beads were placed in a vacuum oven at normal temperature and dried for 24 hours.

(Example 2) Evaluation of X-ray visibility of PLA/PLGA/LPD beads in vivo

In order to evaluate X-ray visibility in vivo, the hepatic artery of a Japanese white rabbit was embolized with the PLA/PLGA/LPD beads, and thereafter, a CT scan was performed. The specific experiment procedure is shown below.

1) By passing the PLA/PLGA/LPD beads (Sample (Form.) 3 (PLA:PLGA:LPD=20:20:60) and Sample 5 (PLA:PLGA:LPD=40:0:60)) through a stainless steel test sieve, beads with a particle size of 300 μm or less were collected.
2) In a solution in which a water-soluble contrast agent (Omunipaque: manufactured by DAIICHI SANKYO COMPANY, LIMITED.) and physiological saline were mixed at a volume ratio of 1:1, the PLA/PLGA/LPD beads were mixed at a volume ratio of the solution:PLA/PLGA/LPD beads of 9: 1.

3) Oxygen and an anesthetic (isoflurane inhalation solution, manufactured by Mylan pharmaceuticals) (2 to 3%) were given to a Japanese white rabbit to make it sleep. Then, after incising the groin, the femoral artery was exposed.

4) Under X-ray fluoroscopy, a catheter was inserted from the femoral artery and approached near the hepatic artery. A small amount of the mixed solution of Omunipaque and physiological saline (volume ratio 1: 1) was injected through the catheter, and angiograph was performed to confirm the position of the artery. Thereafter, the tip of the catheter was inserted into the hepatic artery.

5) Using a syringe, the bead dispersions prepared in 2) was injected into the catheter, and the bead dispersions reached the vicinity of the hepatic artery. At this time, a total of 2 mL was injected at a pace of approximately 1 mL/min, and an X-ray fluoroscopy image was taken during the injection. During bead injection, approximately 5 mL of Omunipaque in total was injected to grasp the embolized state.

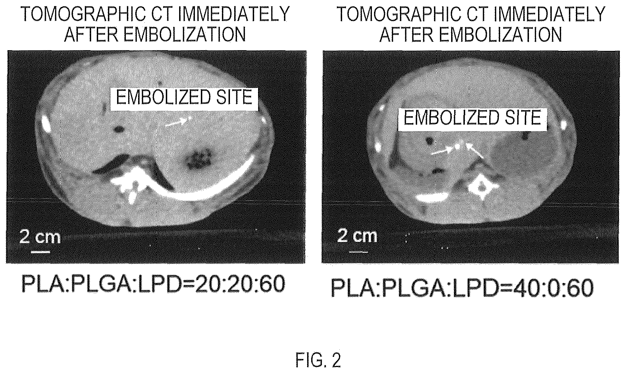

6) After embolization of the hepatic artery, the rabbit was allowed to stand still for an hour to drain the contrast agent from the embolized site, and then the rabbit was euthanized. Thereafter, a CT image was taken. The results are shown in FIG. 2.

As a result, it was found that the hepatic artery was embolized by the PLA/PLGA/LPD beads, and the embolized site could be grasped from the CT image.

(Example 3) Evaluation of biodegradability of PLA/PLGA/LPD beads in vivo

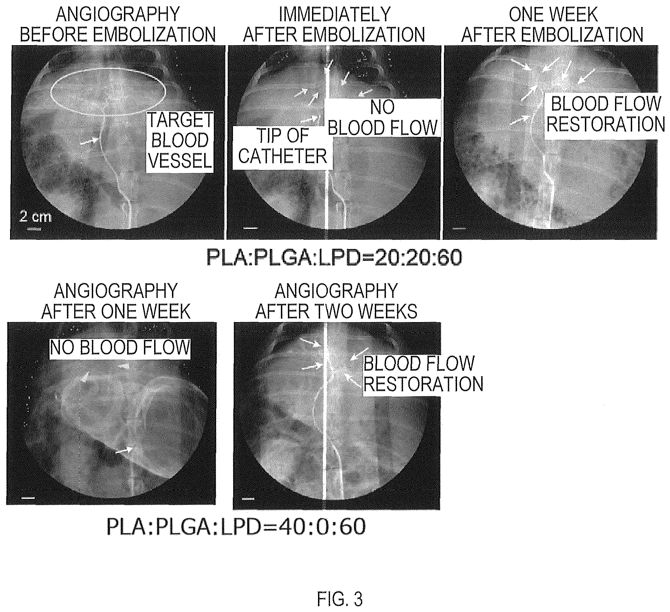

In the same procedure as in Example 2, the hepatic artery of a Japanese white rabbit was embolized with the PLA/PLGA/LPD beads (Sample 3 (PLA:PLGA:LPD=20:20:60) and Sample 5 (PLA:PLGA:LPD=40:0:60)) with a particle size of 300 $\mu$m or less. Thereafter, 0.2 mL of contrast agent was poured into the celiac artery or hepatic artery using a catheter, and angiography was performed. After the angiography, the catheter was removed and the femoral artery was ligated. Thereafter, the exposed part of the groin was sutured and the anesthesia was removed. After one week in the case of Sample 3, after one week and after two weeks in the case of Sample 5, the catheter was approached again to the celiac artery or hepatic artery, and 0.2 mL of contrast agent was poured into the embolized site, and angiography was performed. At this time, the catheter was approached from the femoral artery which had not been ligated during vascular embolization. The results are shown in FIG. 3.

In Sample 3, it was confirmed that the contrast agent did not flow into the hepatic artery and the blood flow was stopped immediately after embolization, whereas the hepatic artery was imaged and the blood flow was restored after one week from embolization. In Sample 5, even after one week from embolization, the blood flow was stopped, but after two weeks from the embolization, the blood flow was restored.

(Example 4) Weight reduction rate measurement of PLA/PLGA/LPD beads In order to evaluate a degradation rate when the PLA/PLGA/LPD beads were degraded with a degrading enzyme, a weight reduction rate of the beads was measured. The specific experiment procedure is shown below.

1) 10 mL of Tris-HCL buffer (pH8.0) (manufactured by FUJIFILM Wako Pure Chemical Corporation) and 1 mL of degrading enzyme Proteinase K (manufactured by TAKARA BIO INC.) were added to 90 mL of purified water.

2) Approximately 50 mg of PLA/PLGA/LPD beads (Sample 3 (PLA:PLGA:LPD=20:20:60), Sample 4 (PLA:PLGA:LPD=30:10:60) and Sample 5 (PLA:PLGA:LPD=40:0:60)) were placed in a 13.5 mL screw bottle, and 5 mL of the solution prepared in 1) was added. Thereafter, the screw bottle was stored in a shaking constant temperature bath at 37°C to degrade the beads with a degrading enzyme.

3) After 3, 7, 14, 21, 28 and 40 days of storage, the screw bottle was taken out of the shaking constant temperature bath, and the degraded beads were taken out from the screw bottle. Using a glass filter paper (GS-25) manufactured by ADVANTEC TOYO KAISHA, LTD., the degraded beads were collected by filtration under reduced pressure. Thereafter, the collected beads were placed in a vacuum oven together with the filter paper, and dried for 24 hours at normal temperature. The weight of the filter paper was measured in advance in a dry state.

4) The weight of the dried beads together with the filter paper was measured to obtain the weight of the degraded beads. Thereafter, a mass reduction rate of the beads was calculated by the following formula (3.1).

[Formula 3.1]

$$Mass\ loss\ (\%) = \frac{W_i - W_d}{W_i} \times 100 \qquad (3.1)$$

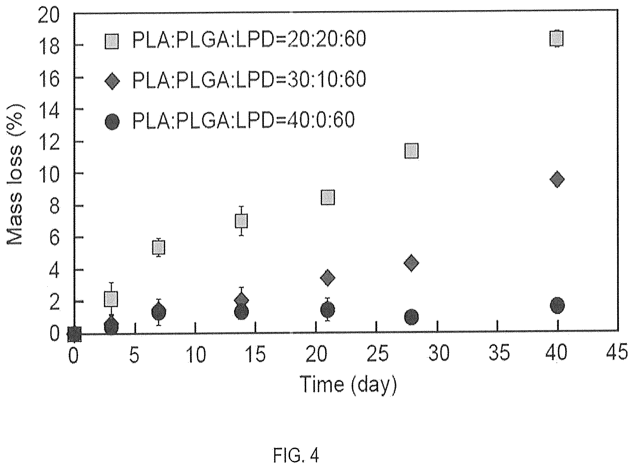

Here, $W_i$ is the mass of the beads before degradation, and $W_d$ is the mass of the degraded beads. The results are shown in FIG. 4.

It was found that, in the PLA/PLGA/LPD beads, an increase in PLGA content increased the weight reduction rate (Mass loss) (the degradation rate increases). In vivo, blood flow restoration is desired in a few days to a week. For that purpose, it was considered that all the materials that make up the beads were not required to be reduced in weight, and the beads only had to be degraded to an extent that the beads disintegrate in the blood flow.

(Example 5) PLA/PLGA/LPD beads molecular weight reduction rate measurement

Molecular weights of the degraded PLA/PLGA/LPD beads (Sample 3 (PLA:PLGA:LPD=20:20:60), Sample 4 (PLA:PLGA:LPD=30:10:60) and Sample 5 (PLA:PLGA:LPD=40:0:60)) collected in Example 4 after 14, 21 and 40 days of storage were measured to evaluate biodegradability of the beads. Approximately 20 mg of the degraded beads was dissolved in 1 mL of chloroform, and the molecular weights of PLA, PLGA and LPD were measured by HPLC (CBM-20A, PID-10A, SPD-20A, DGU-20A3, LC-2040, SIL-20ACHT, column: K-806L (manufactured by Shodex)). The measurement was performed at 25°C, and the flow rate of the mobile phase was 1.0 mL/min. Also, as a standard sample, a polystyrene standard manufactured by Showa Denko K.K. was used. The molecular weight ratio of the measured molecular weights of PLA, PLGA and LPD was measured based on the following formula (3.2).
[Formula 3.2]

$$Molecular\ weight\ ratio = \frac{M_d}{M_i} \qquad (3.2)$$

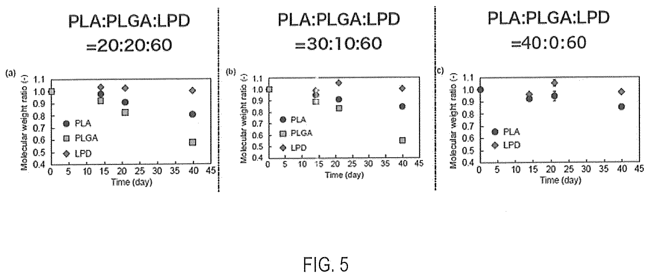

Here, $M_i$ represents the weight average molecular weight of each component in the beads before degradation, and $M_d$ represents the weight average molecular weight of each component in the degraded beads. The molecular weight ratios of each of PLA, PLGA and LPD before and after degradation were calculated. The results are shown in FIG. 5.
After 40 days of storage, the molecular weight ratio of LPD did not substantially decrease, whereas the molecular weight ratios of PLA and PLGA decreased to around 80 to 90% and 55%, respectively. It was found that mainly the PLGA component was degraded.

(Example 6) Composition ratio analysis of PLA, PLGA and LPD in PLA/PLGA/LPD beads

NMR analysis was performed on the PLA/PLGA/LPD beads immediately after adding a solution containing a degrading enzyme (before degradation), and the degraded PLA/PLGA/LPD beads (Sample 3 (PLA:PLGA:LPD=20:20:60), Sample 4 (PLA:PLGA:LPD=30:10:60), and Sample 5 (PLA:PLGA:LPD=40:0:60)) collected in Example 4 after 7, 14, 28 and 40 days of storage, in order to obtain the composition ratio of PLA, PLGA and LPD. Approximately 20 mg of PLA/PLGA/LPD beads was dissolved in 1 mL of deuterated chloroform, and measured by $^1$H-NMR (manufactured by JEOL Ltd., trade name: JNM-ECA500). At this time, the number of scans was 32, and the relaxation time was 5 seconds. Thereafter, from the obtained spectra, the composition ratios of PLA, PLGA and LPD were calculated as follows.

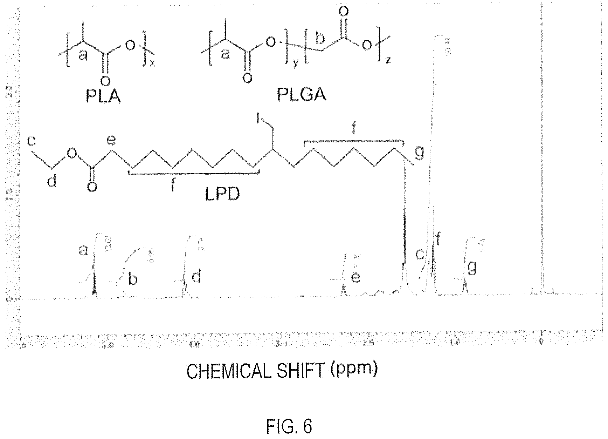

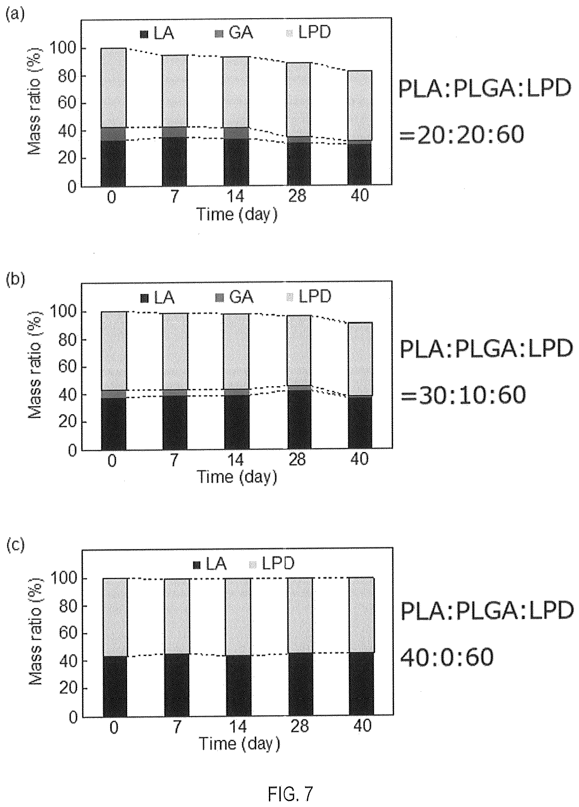

1) For example, when the spectrum as shown in FIG. 6 was obtained, an integrated value was obtained for a peak corresponding to the H element contained in PLA, PLGA and LPD. Peaks (a to g) correspond to portions indicated by subscripts (a to g) in structural formulae of PLA, PLGA and LPD shown in FIG. 6.
2) The integrated value of the peak was divided by the number of H elements corresponding to the peak, and thereafter multiplied by the mass number or molecular weight of the constituent units of the polymer. For example, in case of the spectrum of FIG. 6, the peak specific to glycolic acid (GA) in PLGA is b, and the corresponding H elements are two. Accordingly, when the integrated value of the peak is divided by two, and then multiplied by the mass number 130g/mol of the constituent units of PLGA, 452.4 g/mol is obtained. In the case of PLA, the peak specific to lactic acid (LA) is a, and the corresponding H element is one. Since the integrated value of a includes LA in PLGA, the amount of LA was reduced and then multiplied by the mass number 72g/mol of the constituent unit (since the copolymerization ratio of LA to GA in PLGA is LA:GA=50:50, the integrated value of a derived from PLGA is half the integrated value of b). As for LPD, the same operation was performed based on the peak d, with the molecular weight being 432 g/mol.
3) With respect to PLA, PLGA and LPD, by taking the ratio of the values obtained in 2), the composition ratio of PLA, PLGA and LPD in the beads was obtained. The results were shown in FIG. 7.

Referring to FIG. 7, the mass ratios of LA, GA and LPD before degradation in Sample 3 were 33.2, 9.3 and 57.5%, respectively, and decreased to 29.4, 2.5 and 50.0% after 40 days of storage, respectively. In addition, the masses of GA and LPD in Sample 4 also tended to decrease. The mass ratios before degradation were 37.9, 4.7 and 57.4%, respectively, while, after 40 days of storage, were 36.8, 1.0 and 52.7%, respectively. On the other hand, the mass ratios of LA (PLA) and LPD before degradation in Sample 5 were 43.1 and 56.9%, respectively, but after 40 days of storage, were 44.5 and 54.0%, respectively, and the amount of change in mass ratio between LA and LPD was small. In both Samples 3 and 4, the amount of decrease in mass ratio of LA was smaller than those of GA and LPD, which suggests that the mass loss of the beads of Samples 3 and 4 is caused mainly by hydrolysis of PLGA and leakage of LPD to the outside.

(Example 7) Confirmation of X-ray visibility of PLA/LPD beads

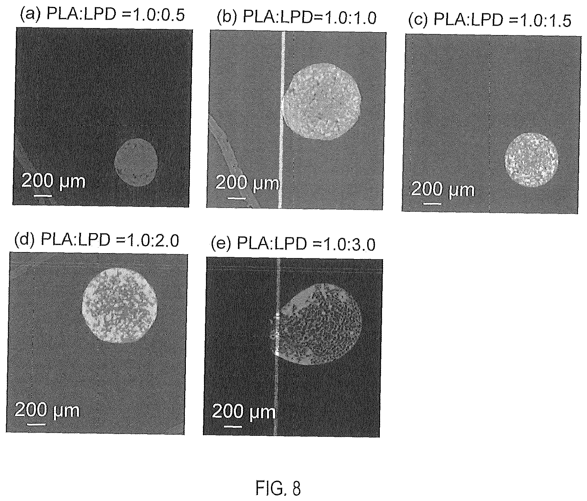

X-ray visibility of the PLA/LPD beads (PLA:LPD=1.0:0.5, PLA:LPD=1.0:1.0, PLA:LPD=1.0:1.5, PLA:LPD=1.0:2.0, PLA:LPD=1.0:3.0) was evaluated using an X-ray micro-CT (manufactured by BRUKER, trade name: SKYSCAN 1272). The beads were placed in a pipette tip and fixed on a stage, and a CT scan was performed with a tube voltage of 50 kV, a tube current of 80 $\mu$A, a focus size of 5 $\mu$m, a measurement field of view of $\phi$5 mm$\times$ 3.8 mm, and a pixel size of 2.2 $\mu$m. The results are shown in FIG. 8. In FIG. 8, white portions indicate good visibility, and black portions indicate poor visibility.

When the amount of LPD charged increased, X-ray visibility also increased. However, when the amount of LPD charged exceeds a certain level, LPD escaped into the PVA aqueous solution, resulting in beads with many voids and low X-ray visibility. Specifically, when 75% of the amount of LPD charged was attempted, LPD flowed out during the bead preparation, and PLA could not enclose LPD. In other words, it is considered that the limit value of the amount of PLA charged is around 70%. Therefore, when PLA is used as the hydrophobic polymer and LPD is used as the oil-based contrast agent, around 4:6 is the optimal mass ratio, and around 3:7 is the limit.

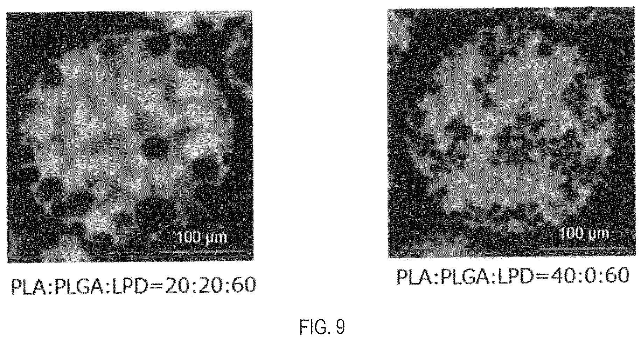

In addition, with respect to the PLA/PLGA/LPD beads (Sample 3 (PLA:PLGA:LPD=20:20:60) and Sample 5 (PLA:PLGA:LPD=40:0:60)), structure analysis of the bead cross section was performed by X-ray micro-CT. The results are shown in FIG. 9.

Voids were observed in the cross section of the beads, and the voids are larger as the amount of PLGA increased. In consideration of the results in vivo together, it is considered that this void structure and the speed of biodegradation of PLGA contribute to degradation of beads to achieve early blood flow restoration under the blood flow.

(Example 8) Shape observation of PLA/PLGA/LPD beads

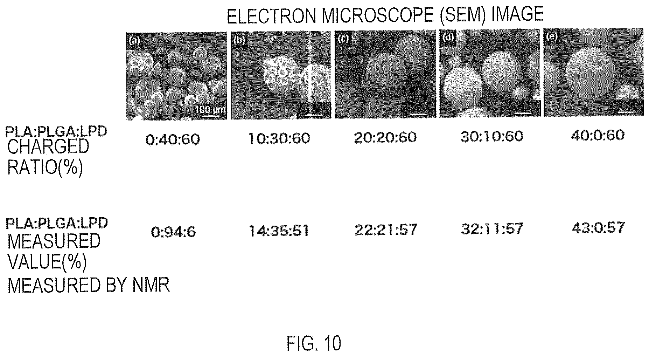

The shape of the PLA/PLGA/LPD beads (Sample 1 (PLA:PLGA:LPD=0:40:60), Sample 2 (PLA:PLGA:LPD=10:30:60), Sample 3 (PLA:PLGA:LPD=20:20:60), Sample 4 (PLA:PLGA:LPD=30:10:60) and Sample 5 (PLA:PLGA:LPD=40:0:60)) was observed using a scanning electron microscope (SEM) manufactured by Thermo Fisher Scientific Inc. Osmium was vapor-deposited on the samples in advance to impart conductivity. The applied voltage during observation was 10 kV. The results were shown in FIG. 10. It was found that when only hydrophilic PLGA was used as the polymer, the particles were hemispherical and could hardly contain LPD (measured value 6% for charged ratio 60%). The composition ratio of PLA, PLGA and LPD in the beads was measured using NMR. Specifically, approximately 20 mg of PLA/PLGA/LPD beads was dissolved in 1 mL of deuterated chloroform, and measurement was performed by $^1$H-NMR (manufactured by JEOL Ltd., trade name: JNM-ECA500). At this time, the number of scans was 32, and the relaxation time was 5 seconds.

Therefore, for example, in order to retain LPD in beads at the mass ratio (measured value) of 60% using PLA as the hydrophobic biodegradable polymer and PLGA as the hydrophilic biodegradable polymer, it is considered necessary to contain PLA at least at the mass ratio of 20% or more.

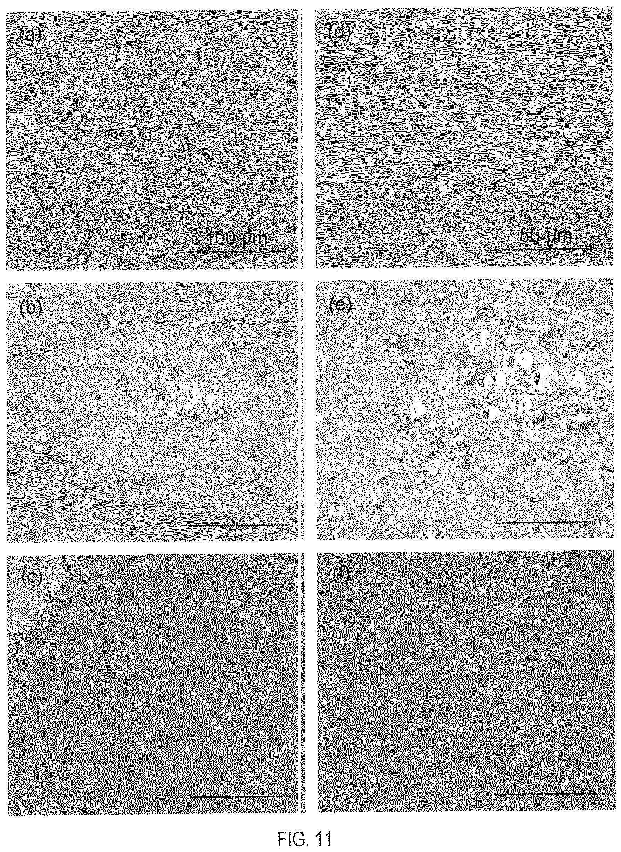

In addition, with respect to the PLA/PLGA/LPD beads (Sample 3 (PLA:PLGA:LPD=20:20:60), Sample 4 (PLA:PLGA:LPD=30:10:60) and Sample 5 (PLA:PLGA:LPD=40:0:60)), when the cross section was observed with an electron microscope after being embedded in epoxy resin, it was found that all the beads had a porous structure inside, as shown in FIG. 11.

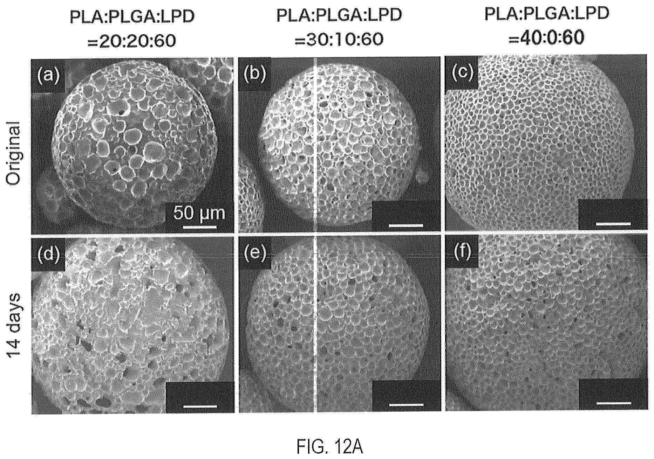

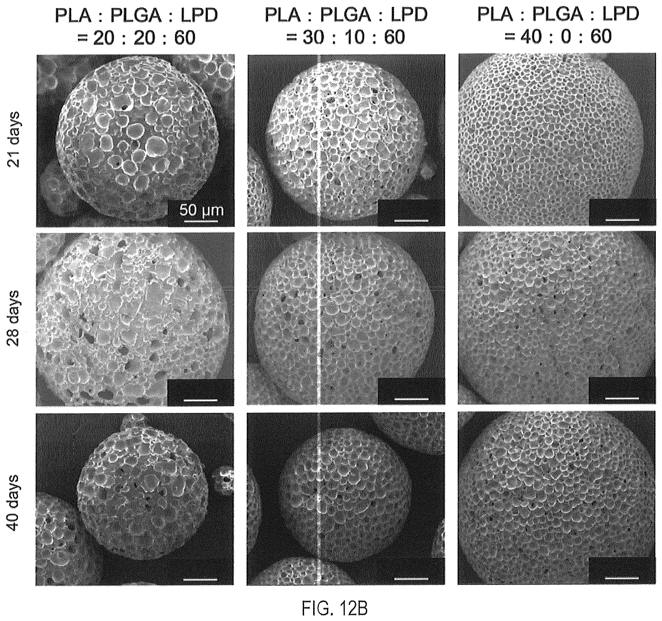

Also, the results of observing the surface structure of the PLA/PLGA/LPD beads (Sample 3 (PLA:PLGA:LPD=20:20:60), Sample 4 (PLA:PLGA:LPD=30:10:60) and Sample 5 (PLA:PLGA:LPD=40:0:60)) were shown in FIGS. 12A and 12B. The upper part of FIG. 12A shows the surface structures of Samples 3, 4 and 5 immediately after adding a solution containing a degrading enzyme (before degradation), and the lower part of FIG. 12A shows the surface structures of Samples 3, 4 and 5 collected in Example 4 after 14 days of storage. The upper part of FIG. 12B shows the surface structures of Samples 3, 4 and 5 collected in Example 4 after 21 days of storage, the middle part of FIG. 12B shows the surface structures of Samples 3, 4 and 5 collected in Example 4 after 28 days of storage, and the lower part of FIG. 12B shows the surface structures of Samples 3, 4 and 5 collected in Example 4 after 40 days of storage.

In the beads of Samples 4 and 5, no significant change was observed before and after degradation. On the other hand, in the beads of Sample 3, traces of erosion on the surface were confirmed. Generally, erosion of biodegradable polymers is caused by chemically breaking the bonds of molecular chains to form oligomers or monomers with small molecular weights, which are then eluted into the external solution, resulting in mass reduction. The erosion observed in the beads of Sample 3 is also considered to have been caused by the molecular chains near the surface becoming low molecular weight due to hydrolysis and being eluted into the immersion solution.

For the size of the beads, the beads (Samples 3 and 4) that contain both the hydrophobic polymer (PLA) and the water-soluble polymer (PLGA) as the polymers were smaller than the beads (Sample 5) that do not contain the hydrophobic polymer.

In addition, both beads had dimples (hemispherical depressions) on the entire surface, and the size of the dimples became smaller as the charged ratio of the hydrophobic polymer (PLA) increased.

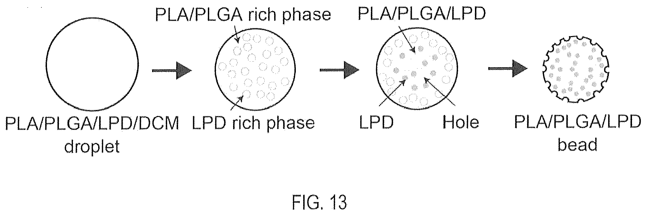

The dimples formed on the bead surface are considered to be caused by phase separation that occurs during formation of the beads in the O/W emulsion method. When PLA/PLGA/LPD/DCM that is a dispersed phase (solution to be dripped) is injected into the PVA aqueous solution that is a continuous phase, PLA/PLGA/LPD/DCM droplets are formed. Thereafter, only DCM gradually volatilizes, and PLA, PLGA and LPD begin to precipitate inside the droplet. At this time, if LPD is present in an amount that cannot be completely mixed with PLA and PLGA, phase separation occurs and an LPD-rich portion is formed (see FIG. 13). Then, when DCM completely volatilizes from the droplet, LPD on the droplet surface is washed into the PVA aqueous solution. It is considered that the traces became the dimples on the bead surface.

(Example 9) Particle size measurement of PLA/LPD beads

In order to measure the particle size of the PLA/LPD beads prepared by different rotation speeds of agitation (500 rpm, 300 rpm, 200 rpm) according to the composition shown in Table 2 below, optical images of the beads were taken using a Leica digital microscope (DVM6). These beads were prepared in the same manner as in Example 1, except for the composition ratio and the rotation speed. Thereafter, the average particle size of 150 or more beads was measured using Motic Image Plus 2.3S software from the images taken at 150x.

[Table 2]

| Form. | Dispersed phase | | | Continuous phase | Agitation rate (rpm) |
| | PLA (g) | LPD (g) | DCM (mL) | PVAaq (w/v%) | |
| --- | --- | --- | --- | --- | --- |
| 1 | | | | | 200 |
| 2 | 0.5 | 1.0 | 20 | 0.5 | 300 |
| 3 | | | | | 500 |

PCL/LPD beads (PCL:LPD=30:70) prepared by a microchannel method were used as a comparative example. Preparation of the PCL/LPD beads by a microchannel was made according to the previously noted publication (Japanese Unexamined Patent Application Publication No. 2018-130322). Specifically, 10 g of polycaprolactone and 10 g of Lipiodol were put into a flask, and a stirring bar of a magnetic stirrer was put therein. A nitrogen atmosphere was maintained in the flask. The flask was heated in a water bath at 80°C. The stirring bar was rotated at 50 rpm to obtain a raw material in molten state containing polycaprolactone and Lipiodol. The raw material was filled into a syringe and maintained at 80°C. The raw material in molten state was extruded from the syringe into the running water at 80°C (flow rate 200 to 500 mL/min) flowing in the pipe, and separated into particles. The volume flow ratio (Qc/Qd) of the continuous phase (water) and the dispersed phase (mixed melt of PCL/LPD) in the microchannel was set to 1500, 2000, 2500 and 3000. The raw material in particulate form was moved into a water tank containing cold water by running water in the pipe. The raw material in particulate form was cooled and solidified in the water tank to obtain beads as the comparative example.

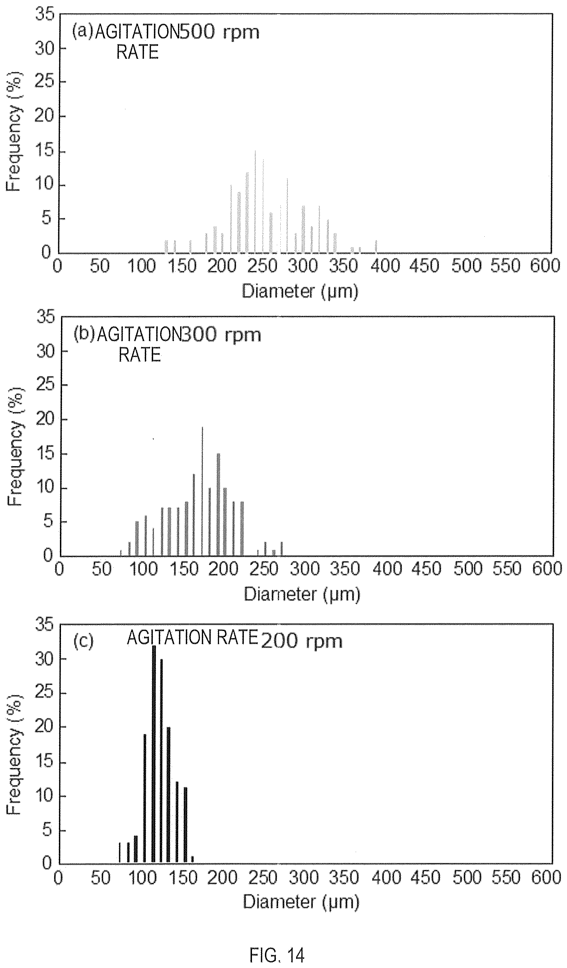

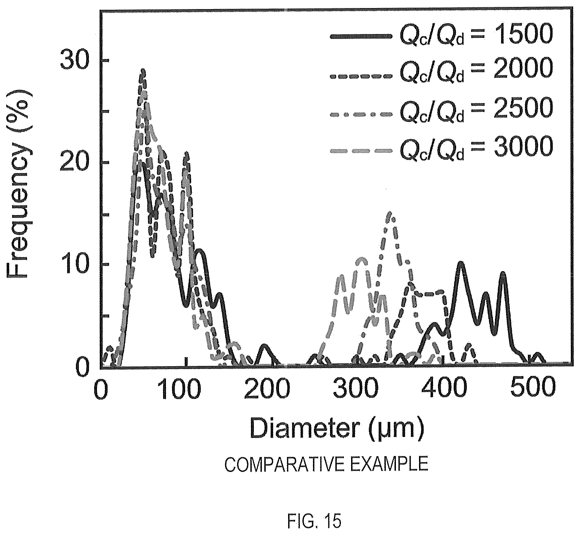

Particle size distributions of the aforementioned various beads are shown in FIGS. 14 and 15.

The PCL/LPD beads prepared by the microchannel method as the comparative example showed a bimodal particle size distribution, whereas the PLA/PLGA/LPD beads prepared by the O/W emulsion method of the present disclosure showed a unimodal (that is, uniform) particle size distribution. In addition, it was found that the O/W emulsion method of the present disclosure can easily reduce the particle size by increasing the agitation rate of the PVA aqueous solution. The average particle size when the agitation rate was 500 rpm was 113.0 $\mu$m, and the standard deviation was 17.6 $\mu$m. The average particle size when the agitation rate was 300 rpm was 161.3 $\mu$m, and the standard deviation was 41.5 $\mu$m. The average particle size when the agitation rate was 200 rpm was 249.7 $\mu$m, and the standard deviation was 51.0 $\mu$m.

(Example 10) Shape observation and composition ratio analysis of PLGA/LPD bead

The PLGA/LPD beads were prepared by the O/W emulsion method. In this Example, PLGA (product name: RESOMER RG502, Mw=7,000 to 17,000, LA:GA=LA:GA=50:50, random copolymer, manufactured by Sigma-Aldrich, or product name: RESOMER RG752H, Mw=4,000 to 15,000, LA:GA=LA:GA=75:25, random copolymer, manufactured by Sigma-Aldrich) were used as the biodegradable polymer. According to the composition shown in Table 3 below, PLGA and LPD were dissolved in DCM. Other steps were carried out by the method described in Example 1. With respect to the PLGA/LPD beads prepared, shape observation by SEM and composition ratio analysis by NMR were carried out according to the method described in Example 8.

[Table 3]

| Sample Name | PLGA (50:50) (g) | PLGA (75:25) (g) | LPD (g) | DCM (mL) |
|---|---|---|---|---|
| Form 1 | 0.4 | 0.0 | | |
| Form 2 | 0.1 | 0.3 | 0.6 | 10 |
| Form 3 | 0.0 | 0.4 | | |

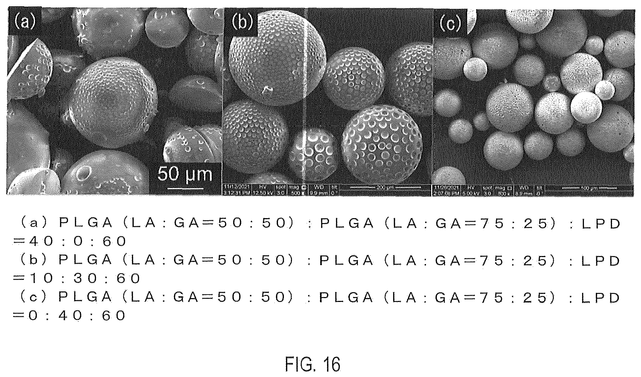

FIG. 16 shows SEM images of Sample 1 (PLGA (LA:GA=50:50):PLGA (LA:GA=75:25):LPD=40:0:60), Sample 2 (PLGA (LA:GA=50:50):PLGA (LA:GA=75:25):LPD=10:30:60), and Sample 3 (PLGA (LA:GA=50:50):PLGA (LA:GA=75:25):LPD=0:40:60). It was found that Sample 1 containing no PLGA (LA:GA=75:25) had a hemispherical shape, whereas Samples 2 and 3 were formed into a spherical shape.

In addition, as shown in Table 4 below with respect to the composition ratio of PLGA and LPD contained in the respective beads, the measured value of LPD for the charged amount of LPD (60% of the whole beads) was 5.6% in Sample 1. It was found that most of the charged amount of LPD had leaked out during preparation of the beads. On the other hand, in Samples 2 and 3, the measured value of LPD was almost the same as the charged amount. It was found that spherical beads sufficiently containing LPD could be prepared by using PLGA (LA:GA=75:25).

[Table 4]

| Sample Name | PLGA (%) | LPD (%) |
|---|---|---|
| Form 1 | 94.4 | 5.6 |
| Form 2 | 44.8 | 55.2 |
| Form 3 | 42.6 | 57.4 |

The beads in Examples 1-9 used PLGA with LA:GA=50:50 as the hydrophilic polymer. However, in this Example, PLGA (LA:GA=75:25), which was made hydrophobic as a whole by increasing the proportion of LA and decreasing the proportion of GA, was used as the biodegradable polymer. This demonstrated that the spherical beads consisting of the biodegradable polymer (especially hydrophobic polymer) and the oil-based contrast agent could be prepared.

PLGA as the hydrophobic polymer is a random copolymer in which the hydrophilic monomer and the hydrophobic monomer are randomly arranged. Therefore, although the polymer as a whole was hydrophobic, PLGA had the property of being easily biodegradable, which suggests that the beads have good biodegradability.

[Effect]

According to Examples described in detail above, an embolic material having good X-ray visibility and biodegradability can be obtained by forming a mixture containing a biodegradable polymer and a liposoluble contrast agent in a hydrophilic liquid.

In addition, by adjusting the weight mixing ratio of the biodegradable polymer and the weight mixing ratio of the liposoluble contrast agent to the biodegradable polymer, an embolic material that has more uniform particle size and a spherical shape, which is useful for selectively embolizing only the spherical target blood vessels, and that exhibits sufficient X-ray visibility can be obtained.

In addition, by adjusting the molar ratio of the hydrophilic monomer and the hydrophobic monomer in a copolymer of the hydrophilic monomer and the hydrophobic monomer, an embolic material having a more uniform particle size, better formability, X-ray visibility and biodegradability can be obtained even if one type of polymer is used.

**Claims**

1.  An embolic material produced by a production method comprising a step for dispersing a mixture comprising a biodegradable polymer, an oil-based contrast agent and a solvent in a hydrophilic liquid,

the biodegradable polymer comprising a hydrophobic biodegradable polymer and a hydrophilic biodegradable polymer,

the hydrophobic biodegradable polymer consisting of one or more selected from polylactic acid, PLA, and polycaprolactone, PCL,

the hydrophilic biodegradable polymer consisting of one or more selected from poly(lactic-co-glycolic acid), PLGA, and caprolactone-co-glycolic acid, PCGA, a weight mixing ratio of the oil-based contrast agent to the biodegradable polymer being 1.0 to 2.0, and

a content of the hydrophobic biodegradable polymer being 20 to 40% by mass when a content of the embolic material is 100% by mass.

2. The embolic material according to claim 1, wherein the step is performed by supplying the mixture to the hydrophilic liquid while agitating the hydrophilic liquid, thereby dispersing the mixture in the hydrophilic liquid.

3. An embolic material in particulate form produced by a production method comprising a step for dispersing a mixture comprising a biodegradable polymer, an oil-based contrast agent and a solvent in a hydrophilic liquid, the biodegradable polymer comprising a hydrophobic biodegradable polymer and a hydrophilic biodegradable polymer,

wherein the hydrophobic biodegradable polymer and the hydrophilic biodegradable polymer are copolymers having the same hydrophilic monomer and hydrophobic monomer as constituent units, and

wherein the hydrophobic biodegradable polymer and the hydrophilic biodegradable polymer are poly(lactic-co-glycolic acid), PLGA.

4. The embolic material according to claim 3, wherein an amount of lactic acid in poly(lactic-co-glycolic acid), PLGA, that is the hydrophobic biodegradable polymer is greater than an amount of glycolic acid.

5. The embolic material according to claim 3 or 4, wherein an amount of glycolic acid in poly(lactic-co-glycolic acid), PLGA, that is the hydrophilic biodegradable polymer is equal to or greater than an amount of lactic acid.

6. The embolic material according to any one of claims 3 to 5, wherein a weight mixing ratio of the oil-based contrast agent to the biodegradable polymer is 1.0 to 2.0.

7. The embolic material according to any one of claims 3 to 6, wherein the step is performed by supplying the mixture to the hydrophilic liquid while agitating the hydrophilic liquid, thereby dispersing the mixture in the hydrophilic liquid.

8. A production method of an embolic material comprising a step for dispersing a mixture comprising a biodegradable polymer, an oil-based contrast agent and a solvent in a hydrophilic liquid,

the biodegradable polymer comprising a hydrophobic biodegradable polymer and a hydrophilic biodegradable polymer,

the hydrophobic biodegradable polymer consisting of one or more selected from polylactic acid, PLA, and polycaprolactone, PCL,

the hydrophilic biodegradable polymer consisting of one or more selected from poly(lactic-co-glycolic acid), PLGA, and caprolactone-co-glycolic acid, PCGA,

a weight mixing ratio of the oil-based contrast agent to the biodegradable polymer being 1.0 to 2.0, and

a content of the hydrophobic biodegradable polymer being 20 to 40% by mass when a content of the embolic material is 100% by mass.

**Patentansprüche**

1. Emboliematerial, hergestellt durch ein Herstellungsverfahren, das einen Schritt zum Dispergieren eines Gemischs umfasst, das ein biologisch abbaubares Polymer, ein Kontrastmittel auf Ölbasis und ein Lösungsmittel in einer hydrophilen Flüssigkeit umfasst,

wobei das biologisch abbaubare Polymer ein hydrophobes biologisch abbaubares Polymer und ein hydrophiles biologisch abbaubares Polymer umfasst,

wobei das hydrophobe biologisch abbaubare Polymer aus einem oder mehreren ausgewählt aus Polymilchsäure, PLA, und Polycaprolacton, PCL, besteht,
wobei das hydrophile biologisch abbaubare Polymer aus einem oder mehreren ausgewählt aus Poly(milch-co-glycolsäure), PLGA, und Caprolacton-co-glycolsäure, PCGA, besteht, wobei ein Gewichtsmischungsverhältnis des Kontrastmittels auf Ölbasis zu dem biologisch abbaubaren Polymer 1,0 bis 2,0 beträgt, und
wobei ein Gehalt des hydrophoben biologisch abbaubaren Polymers 20 bis 40 Massen-% beträgt, wenn ein Gehalt des Emboliematerials 100 Massen-% beträgt.

2. Emboliematerial nach Anspruch 1, wobei der Schritt durch Liefern des Gemischs an die hydrophile Flüssigkeit durchgeführt wird, während die hydrophile Flüssigkeit gerührt wird, wodurch das Gemisch in der hydrophilen Flüssigkeit dispergiert wird.

3. Emboliematerial in Partikulatform, hergestellt durch ein Herstellungsverfahren, das einen Schritt zum Dispergieren eines Gemischs umfasst, das ein biologisch abbaubares Polymer, ein Kontrastmittel auf Ölbasis und ein Lösungsmittel in einer hydrophilen Flüssigkeit umfasst, wobei das biologisch abbaubare Polymer ein hydrophobes biologisch abbaubares Polymer und ein hydrophiles biologisch abbaubares Polymer umfasst,

wobei das hydrophobe biologisch abbaubare Polymer und das hydrophile biologisch abbaubare Polymer Copolymere sind, die das gleiche hydrophile Monomer und hydrophobe Monomer als Bestandteileinheiten aufweisen, und
wobei das hydrophobe biologisch abbaubare Polymer und das hydrophile biologisch abbaubare Polymer Poly(milch-co-glycolsäure), PLGA, sind.

4. Emboliematerial nach Anspruch 3, wobei eine Menge an Milchsäure in Poly(milch-co-glycolsäure), PLGA, das heißt dem hydrophoben biologisch abbaubaren Polymer, größer als eine Menge an Glycolsäure ist.

5. Emboliematerial nach Anspruch 3 oder 4, wobei eine Menge an Glycolsäure in Poly(milch-co-glycolsäure), PLGA, das heißt dem hydrophilen biologisch abbaubaren Polymer, gleich einer oder größer als eine Menge an Milchsäure ist.

6. Emboliematerial nach einem der Ansprüche 3 bis 5, wobei ein Gewichtsmischungsverhältnis des Kontrastmittels auf Ölbasis zu dem biologisch abbaubaren Polymer 1,0 bis 2,0 beträgt.

7. Emboliematerial nach einem der Ansprüche 3 bis 6, wobei der Schritt durch Liefern des Gemischs an die hydrophile Flüssigkeit durchgeführt wird, während die hydrophile Flüssigkeit gerührt wird, wodurch das Gemisch in der hydrophilen Flüssigkeit dispergiert wird.

8. Herstellungsverfahren für ein Emboliematerial, das einen Schritt zum Dispergieren eines Gemischs umfasst, das ein biologisch abbaubares Polymer, ein Kontrastmittel auf Ölbasis und ein Lösungsmittel in einer hydrophilen Flüssigkeit umfasst,

wobei das biologisch abbaubare Polymer ein hydrophobes biologisch abbaubares Polymer und ein hydrophiles biologisch abbaubares Polymer umfasst,
wobei das hydrophobe biologisch abbaubare Polymer aus einem oder mehreren ausgewählt aus Polymilchsäure, PLA, und Polycaprolacton, PCL, besteht,
wobei das hydrophile biologisch abbaubare Polymer aus einem oder mehreren ausgewählt aus Poly(milch-co-glycolsäure), PLGA, und Caprolacton-co-glycolsäure, PCGA, besteht,
wobei ein Gewichtsmischungsverhältnis des Kontrastmittels auf Ölbasis zu dem biologisch abbaubaren Polymer 1,0 bis 2,0 beträgt, und
wobei ein Gehalt des hydrophoben biologisch abbaubaren Polymers 20 bis 40 Massen-% beträgt, wenn ein Gehalt des Emboliematerials 100 Massen-% beträgt.

**Revendications**

1. Matériau embolique produit par un procédé de production comprenant une étape de dispersion d'un mélange comprenant un polymère biodégradable, un agent de contraste à base d'huile et un solvant dans un liquide hydrophile,

le polymère biodégradable comprenant un polymère biodégradable hydrophobe et un polymère biodégradable hydrophile,

le polymère biodégradable hydrophobe étant constitué de l'un ou plusieurs choisis parmi l'acide polylactique, PLA et la polycaprolactone, PCL,

le polymère biodégradable hydrophile étant constitué de l'un ou plusieurs choisis parmi le poly(acide lactique-co-glycolique), PLGA, et le caprolactone-co-acide glycolique, PCGA, un rapport de mélange en poids de l'agent de contraste à base d'huile au polymère biodégradable étant de 1,0 à 2,0, et

une teneur du polymère biodégradable hydrophobe étant de 20 à 40 % en masse lorsqu'une teneur du matériau embolique est de 100 % en masse.

2. Matériau embolique selon la revendication 1, dans lequel l'étape est réalisée en fournissant le mélange au liquide hydrophile tout en agitant le liquide hydrophile, dispersant ainsi le mélange dans le liquide hydrophile.

3. Matériau embolique sous forme particulaire produit par un procédé de production comprenant une étape de dispersion d'un mélange comprenant un polymère biodégradable, un agent de contraste à base d'huile et un solvant dans un liquide hydrophile, le polymère biodégradable comprenant un polymère biodégradable hydrophobe et un polymère biodégradable hydrophile,

dans lequel le polymère biodégradable hydrophobe et le polymère biodégradable hydrophile sont des copolymères ayant le même monomère hydrophile et le même monomère hydrophobe comme unités constitutives, et dans lequel le polymère biodégradable hydrophobe et le polymère biodégradable hydrophile sont le poly(acide lactique-co-glycolique), PLGA.

4. Matériau embolique selon la revendication 3, dans lequel une quantité d'acide lactique dans le poly(acide lactique-co-glycolique), PLGA, qui est le polymère biodégradable hydrophobe est supérieure à une quantité d'acide glycolique.

5. Matériau embolique selon l'une des revendications 3 ou 4, dans lequel une quantité d'acide glycolique dans le poly(acide lactique-co-glycolique), PLGA, qui est le polymère biodégradable hydrophile est supérieure ou égale à une quantité d'acide lactique.

6. Matériau embolique selon l'une quelconque des revendications 3 à 5, dans lequel un rapport de mélange en poids de l'agent de contraste à base d'huile au polymère biodégradable est de 1,0 à 2,0.

7. Matériau embolique selon l'une quelconque des revendications 3 à 6, dans lequel l'étape est réalisée en fournissant le mélange au liquide hydrophile tout en agitant le liquide hydrophile, dispersant ainsi le mélange dans le liquide hydrophile.

8. Procédé de production d'un matériau embolique comprenant une étape de dispersion d'un mélange comprenant un polymère biodégradable, un agent de contraste à base d'huile et un solvant dans un liquide hydrophile,

le polymère biodégradable comprenant un polymère biodégradable hydrophobe et un polymère biodégradable hydrophile,

le polymère biodégradable hydrophobe étant constitué de l'un ou plusieurs choisis parmi l'acide polylactique, PLA et la polycaprolactone, PCL,

le polymère biodégradable hydrophile étant constitué de l'un ou plusieurs choisis parmi le poly(acide lactique-co-glycolique), PLGA, et le caprolactone-co-acide glycolique, PCGA,

un rapport de mélange en poids de l'agent de contraste à base d'huile au polymère biodégradable étant de 1,0 à 2,0, et

une teneur du polymère biodégradable hydrophobe étant de 20 à 40 % en masse lorsqu'une teneur du matériau embolique est de 100 % en masse.

FIG. 1

TOMOGRAPHIC CT IMMEDIATELY AFTER EMBOLIZATION

EMBOLIZED SITE

2 cm

PLA:PLGA:LPD=20:20:60

TOMOGRAPHIC CT IMMEDIATELY AFTER EMBOLIZATION

EMBOLIZED SITE

2 cm

PLA:PLGA:LPD=40:0:60

FIG. 2

ANGIOGRAPHY
BEFORE EMBOLIZATION

IMMEDIATELY
AFTER EMBOLIZATION

ONE WEEK
AFTER EMBOLIZATION

TARGET
BLOOD
VESSEL

2 cm

TIP OF
CATHETER

NO
BLOOD FLOW

BLOOD FLOW
RESTORATION

PLA:PLGA:LPD=20:20:60

ANGIOGRAPHY
AFTER ONE WEEK

ANGIOGRAPHY
AFTER TWO WEEKS

NO BLOOD FLOW

BLOOD FLOW
RESTORATION

PLA:PLGA:LPD=40:0:60

FIG. 3

FIG. 4

FIG. 5

CHEMICAL SHIFT (ppm)

FIG. 6

FIG. 7

(a) PLA:LPD =1.0:0.5 (b) PLA:LPD=1.0:1.0 (c) PLA:LPD =1.0:1.5

(d) PLA:LPD =1.0:2.0 (e) PLA:LPD =1.0:3.0

FIG. 8

PLA:PLGA:LPD=20:20:60

PLA:PLGA:LPD=40:0:60

FIG. 9

ELECTRON MICROSCOPE (SEM) IMAGE

| PLA:PLGA:LPD CHARGED RATIO(%) | 0:40:60 | 10:30:60 | 20:20:60 | 30:10:60 | 40:0:60 |

| PLA:PLGA:LPD MEASURED VALUE(%) MEASURED BY NMR | 0:94:6 | 14:35:51 | 22:21:57 | 32:11:57 | 43:0:57 |

FIG. 10

FIG. 11

FIG. 12A

FIG. 12B

PLA/PLGA rich phase  PLA/PLGA/LPD

PLA/PLGA/LPD/DCM  LPD rich phase    LPD        Hole     PLA/PLGA/LPD
droplet                                                  bead

FIG. 13

FIG. 14

COMPARATIVE EXAMPLE

FIG. 15

(a) ＰＬＧＡ（ＬＡ：ＧＡ＝５０：５０）：ＰＬＧＡ（ＬＡ：ＧＡ＝７５：２５）：ＬＰＤ
＝４０：０：６０
(b) ＰＬＧＡ（ＬＡ：ＧＡ＝５０：５０）：ＰＬＧＡ（ＬＡ：ＧＡ＝７５：２５）：ＬＰＤ
＝１０：３０：６０
(c) ＰＬＧＡ（ＬＡ：ＧＡ＝５０：５０）：ＰＬＧＡ（ＬＡ：ＧＡ＝７５：２５）：ＬＰＤ
＝０：４０：６０

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021008824 A
- JP 2018130322 A

- JP 2004167229 A

**Non-patent literature cited in the description**

- **R. DURAN et al.** *Theranostics*, 2016, vol. 6, 28-39
- **N. RICHARD et al.** *Case Reports in Gastroenterology*, 2018, vol. 12 (2), 352-9

- **A. TORO et al.** *Case Reports in Surgery*, 2015
- **Y. OKUNO et al.** *J. Vasc. Interv. Radiol.*, 2013, vol. 24, 787-792